(19) Europäisches Patentamt
European Patent Office
Office européen des brevets

(11) EP 3 954 399 B1

(12) **EUROPEAN PATENT SPECIFICATION**

(45) Date of publication and mention of the grant of the patent:
**15.11.2023 Bulletin 2023/46**

(21) Application number: **21784358.0**

(22) Date of filing: **07.04.2021**

(51) International Patent Classification (IPC):
***A61L 24/10*** *(2006.01)* ***A61B 17/12*** *(2006.01)*

(52) Cooperative Patent Classification (CPC):
(C-Sets available)
**A61L 24/0036; A61L 24/001; A61L 24/0015; A61L 24/043; A61L 24/104;** A61L 2300/216; A61L 2300/23; A61L 2300/416; A61L 2300/45

(Cont.)

(86) International application number:
**PCT/KR2021/004336**

(87) International publication number:
**WO 2021/206440 (14.10.2021 Gazette 2021/41)**

(54) **MICROBEADS FOR EMBOLIZATION AND COMPOSITION FOR TREATING PROLIFERATIVE DISEASES**

MIKROKÜGELCHEN ZUR EMBOLISATION UND ZUSAMMENSETZUNG ZUR BEHANDLUNG VON PROLIFERATIVEN KRANKHEITEN

MICROBILLES POUR EMBOLISATION ET COMPOSITION POUR TRAITER DES MALADIES PROLIFÉRATIVES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.04.2020 KR 20200043611**
**19.03.2021 KR 20210035678**

(43) Date of publication of application:
**16.02.2022 Bulletin 2022/07**

(73) Proprietor: **Plmicromed Co., Ltd.**
**Yangsan-si, Gyeongsangnam-do 50620 (KR)**

(72) Inventor: **KIM, Gwang Suk**
**Yangsan-si, Gyeongsangnam-do 50594 (KR)**

(74) Representative: **Manitz Finsterwald**
**Patent- und Rechtsanwaltspartnerschaft mbB**
**Martin-Greif-Strasse 1**
**80336 München (DE)**

(56) References cited:
**EP-A1- 2 351 779**
**CN-A- 110 251 457**
**JP-A- 2012 507 562**
**KR-A- 20070 046 850**
**KR-A- 20110 119 612**

- **DOBRINCIC ANA ET AL: "Advanced Technologies for the Extraction of Marine Brown Algal Polysaccharides", MARINE DRUGS, vol. 18, no. 3, 1 January 2020 (2020-01-01), pages 1-29, XP055856321, DOI: 10.3390/md18030168**
- **Dobrin i Ana; Balbino Sandra; Zori Zoran; Pedisi Sandra; Bursa Kova evi Danijela; Elez Garofuli Ivona; Dragovi -Uzelac Veric: "Advanced Technologies for the Extraction of Marine Brown Algal Polysaccharides", Marine Drugs, vol. 18, no. 3, 168, 18 March 2020 (2020-03-18), pages 1-29, XP055856321, DOI: 10.3390/md18030168**
- **Ko, Jung-A , Oh, Youn-Sung , Park, Hyun-Jin: "Preparation and Characterization of Aminated Gelatin-Fucoidan Microparticles", Korean Society of Food Science and Technology, vol. 44, no. 2, 1 January 2012 (2012-01-01), pages 191-195, XP055856326, ISSN: 0367-6293, DOI: 10.9721/kjfst.2012.44.2.191**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• **Rassu Giovanna, Salis Andrea, Porcu Elena Piera, Giunchedi Paolo, Roldo Marta, Gavini Elisabetta: "Composite chitosan/alginate hydrogel for controlled release of deferoxamine: A system to potentially treat iron dysregulation diseases", Carbohydrate Polymers, vol. 136, 1 January 2016 (2016-01-01), pages 1338-1347, XP055856331, GB ISSN: 0144-8617, DOI: 10.1016/j.carbpol.2015.10.048**

(52) Cooperative Patent Classification (CPC): (Cont.)

C-Sets
**A61L 24/043, C08L 5/00;**
**A61L 24/043, C08L 89/06;**
**A61L 24/043, C08L 99/00**

## Description

### TECHNICAL FIELD

The present disclosure relates to an embolic microbead and a composition for treating proliferative diseases.

### BACKGROUND

Embolization or embolotherapy is a procedure that treats a tumor by injecting a specific substance into a blood vessel to block blood that carries oxygen and nutrients to a tumor tissue.

A general substance for embolization needs to be biocompatible, hydrophilic, nontoxic and biodegradable. Therefore, microparticles such as chitosan, starch, gelatin, albumin and sodium alginate are mainly being researched as embolic materials.

In particular, as for gelatin, a hemostatic gelatin product developed using 1960's mammal (cow, pig)-derived gelatin is used. Since a porous sponge sheet type gelatin is processed by cutting or mixing, it has irregular shapes. Also, gelatin granules stick together during microcatheter embolization. Accordingly, it is hard to expect the embolic effect as desired by clinicians.

Meanwhile, as for a patient with liver cancer who cannot undergo surgical resection, local treatments such as transcatheter arterial chemoembolization (TACE), percutaneous ethanol injection therapy (PEIT) and radiofrequency thermal ablation (RFA) are being used. In particular, the transcatheter arterial chemoembolization is an (embolization) procedure that blocks a blood vessel by injecting an anticancer agent and lipiodol into an artery through which nutrients and oxygen are supplied to a tumor, and induces anticancer effect and ischaemic necrosis of the tumor and thus is effective in treating liver cancer. However, when complications are caused by an embolization procedure or drugs, an adverse effect of the treatment may occur. It has been reported by researchers that postembolization syndrome, which is one of the systemic complications, occurs with a frequency of from about 60% to about 80%. The postembolization syndrome is the most common complications occurring after the transcatheter arterial chemoembolization, and may include vomiting, appetite loss, fever and abdominal pain. The quality of life of patients has deteriorated due to these complications, which may cause sometimes the patients to stop taking their treatment even if the treatment is effective.

Recently, "microsphere embolization" developed from transcatheter arterial chemoembolization has been performed using a large amount of anticancer agent. Thus, it is possible to effectively remove cancer cells. The microsphere embolization is a procedure that selectively administers embolic drug-eluting beads, which are microspheres that keep an anticancer agent and gradually elute the anticancer agent for hours, to cancer cells. The microsphere embolization enables a high-concentration anticancer drug to be kept in tumor blood vessels and thus results in a high treatment reaction rate and a high suppression effect. However, drug-eluting beads are composed of semi-permanent embolic materials instead of temporary embolic materials and thus may cause blockages in the embolized hepatic artery. Therefore, the microsphere embolization may not be repeatedly performed and may cause a high likelihood of chronic inflammatory response.

[Related prior art] KR 10-2001-0068458 A

EP 2 351 779 discloses embolic microspheres formed by crosslinking gelatin and a copolymer comprising a N-tris-hydroxymethyl methylacrylamide monomer unit, a diethylaminoethylacrylamide monomer unit and a N,N-methylene-bisacrylamide monomer unit. The 77microsphere may have drug loading characteristics. For example, the microsphere can be loaded with doxorubucin which is an anticancer agent.

### DISCLOSURE OF THE INVENTION

### PROBLEMS TO BE SOLVED BY THE INVENTION

The present disclosure relates to an embolic microbead that have an improved iron adsorption rate and can effectively suppress cancer cell proliferation, and a composition for treating proliferative diseases.

### MEANS FOR SOLVING THE PROBLEMS

An embolic microbead according to an exemplary embodiment of the present disclosure includes a biocompatible polymer, and the biocompatible polymer includes a gelatin cross-linked with an iron adsorption block, wherein the iron adsorption block includes a fucoidan and/or a polyphenol.

In an exemplary embodiment of the present disclosure, the polyphenol may be a biodegradable anionic polyphenol, and the polyphenol may include at least one selected from tannin, gallic acid, tannic acid, catechin, epicatechin, epigallocatechin gallate, quercetin, resveratrol, isoflavone, flavonoids, and polyphenols derived from seaweeds.

In an exemplary embodiment of the present disclosure, the gelatin may be an aminated gelatin, and the embolic microbead may include cross-linking of a cationic aminated gelatin and an anionic fucodian.

In an exemplary embodiment of the present disclosure, a weight ratio of the gelatin : the iron adsorption block may be about 4:1 to about 10:1.

In an exemplary embodiment of the present disclosure, the gelatin may include a first gelatin having a first average molecular weight; and a second gelatin having a second average molecular weight smaller than the first average molecular weight.

In an exemplary embodiment of the present disclosure, an iron adsorption rate after 24 hours(IAd24) based on 100 mg of the embolic microbead, represented by the following Formula 1, may be about 5 %/100 mg or more:

[Formula 1] $$IAd24 = \frac{(IC0-IC24)}{IC0} \times 100$$ ;

in the Formula 1, the IC0 is an initial molar concentration of a $FeCl_3$ aqueous solution, and the IC24 is a molar concentration of an iron ion of the $FeCl_3$ aqueous solution left for 24 hours after adding the embolic microbead to the $FeCl_3$ aqueous solution.

In an exemplary embodiment of the present disclosure, an iron adsorption rate after 48 hours(IAd48) based on 100 mg of the embolic microbead, represented by the following Formula 2, may be about 5 %/100 mg or more:

[Formula 2] $$IAd48 = \frac{(IC0 - IC48)}{IC0} \times 100$$ ;

in the Formula 2, the IC0 is an initial molar concentration of a $FeCl_3$ aqueous solution, and the IC48 is a molar concentration of an iron ion of the $FeCl_3$ aqueous solution left for 48 hours after adding the embolic microbead to the $FeCl_3$ aqueous solution.

In an exemplary embodiment of the present disclosure, an embolic microbead may include a biocompatible polymer, the biocompatible polymer may include a gelatin cross-linked with an iron adsorption block, wherein the iron adsorption block includes a fucoidan and/or a polyphenol, and the embolic microbead may be combined with an anticancer drug.

In an exemplary embodiment of the present disclosure, the anticancer drug may be selected from doxorubicin, irinotecan, gemcitabine, oxaliplatin and docetaxel.

In an exemplary embodiment of the present disclosure, a combining rate with the anticancer drug after 1 hour(AAd) based on 100 mg of the embolic microbead, represented by the following Formula 6, may be about 10 %/100 mg or more:

[Formula 6] $$AAd = \frac{(AC0-AC1)}{AC0} \times 100$$ ;

in the Formula 6, the AC0 is an initial mg/mℓ concentration of an anticancer drug solution, and the AC1 is a mg/mℓ concentration of the anticancer drug of the anticancer drug solution left for 1 hour after adding the embolic microbead to the anticancer drug solution.

A composition for treating proliferative diseases according to an exemplary embodiment of the present disclosure includes an embolic microbead, the embolic microbead includes a gelatin cross-linked with an iron adsorption block, wherein the iron adsorption block includes a fucoidan and/or a polyphenol, and the embolic microbead is combined with an iron component and/or an anticancer drug.

In an exemplary embodiment of the present disclosure, the proliferative diseases may include at least one selected from neovascularization, tumor, cancer, bone diseases, fibroplasia disorders, statomegaly, renal cyst, and atherosclerosis.

In an exemplary embodiment of the present disclosure, the cancer may include at least one selected from solid tumor, hematologic malignancy, colorectal cancer, uterine cancer, uterine myoma, meningioma, lung cancer, small cell lung cancer, non-small cell lung cancer, gastrointestinal cancer, bowel cancer, breast cancer, ovarian cancer, prostate

cancer, testicular cancer, liver cancer, renal cancer, bladder cancer, pancreatic cancer, sarcoma, osteosarcoma, kaposi's sarcoma and melanoma.

Effects of the invention

An embolic microbead according to an exemplary embodiment of the present disclosure includes a polymer containing an iron adsorption block capable of adsorbing iron. Therefore, the embolic microbead according to an exemplary embodiment of the present disclosure can effectively adsorb iron.

Also, the embolic microbead according to an exemplary embodiment of the present disclosure has a high iron adsorption rate after 24 hours and a high iron adsorption rate after 48 hours. Therefore, the embolic microbead according to an exemplary embodiment of the present disclosureis capable of fast iron adsorption.

Therefore, the embolic microbead according to an exemplary embodiment of the present disclosureis injected into a human body, particularly the liver carotid artery so that blood flow to cancer cells or the like can be blocked and the supply of iron ions, which are required for existence or proliferation of cancer cells, to the cancer cells can be blocked.

Therefore, the embolic microbead according to an exemplary embodiment of the present disclosure can effectively necrotize cancer cells by blocking a blood vessel.

Also, the embolic microbead according to an exemplary embodiment of the present disclosure can effectively adsorb iron ions that are eluted when cancer cells or the like are necrotized.

Therefore, the embolic microbead according to an exemplary embodiment of the present disclosure can effectively suppress regrowth of cancer cells.

The embolic microbead according to an exemplary embodiment of the present disclosure can be very effectively used for treating cancers, such as liver cancer, and treating proliferative diseases.

A low molecularized embolic microbead and a composition for treating proliferative diseases including the same according to an exemplary embodiment of the present disclosure have a low viscosity and thus can pass through a microcatheter. Also, the embolic microbead and the composition are safely absorbed into blood vessels within from about 1 day to about 10 days, or within from about 2 days to about 3 days and thus facilitates recanalization and reoperation, and can maintain and protect normal blood vessels during reoperation. Further, the low molecular weight embolic microbead and the composition for treating proliferative diseases including the same according to an exemplary embodiment of the present disclosure can selectively lesional blood vessels of a tumor while protecting normal blood vessels and can reduce side effects, such as postembolization syndrome or the like, as well as side effects caused by inflammatory response.

The embolic microbead and the composition for treating proliferative diseases including the same according to an exemplary embodiment of the present disclosure are gradually eluted into blood as gelatin beads coupled to the iron adsorption block are gradually absorbed into the body. Therefore, the embolic microbead and the composition for treating proliferative diseases including the same according to an exemplary embodiment of the present disclosure have a safe anticancer effect selectively to cancer cells and cause insignificant side effects. Therefore, the embolic microbead and the composition for treating proliferative diseases including the same according to an exemplary embodiment of the present disclosure can be used for treating various solid cancers that proliferate by forming new blood vessels.

## BRIEF DESCRIPTION OF THE DRAWINGS

**FIG. 1** is a diagram showing a process of preparing an embolic microbead according to an example.

**FIG. 2** shows a calibration curve for the absorbance of light of a wavelength of 550 nm, which is the basis of calculation of a doxorubicin conjugation rate according to an example, against the concentration of an embolic microbead according to the present disclosure.

## BEST MODE FOR CARRYING OUT THE INVENTION

Hereinafter, the present disclosure will be described in more detail with reference to exemplary embodiments.

An embolic microbead according to an exemplary embodiment of the present disclosure includes a biocompatible polymer. An embolic microbead according to an exemplary embodiment of the present disclosure may be composed of the biocompatible polymer.

The embolic microbead according to an exemplary embodiment of the present disclosure may include the biocompatible polymer in the amount of about 80 parts or more by weight based on 100 parts by weight of the microbead. The embolic microbead according to an exemplary embodiment of the present disclosure may include the biocompatible polymer in the amount of about 90 parts or more by weight based on 100 parts by weight of the microbead. The embolic

microbead according to an exemplary embodiment of the present disclosure may include the biocompatible polymer in the amount of about 95 parts or more by weight based on 100 parts by weight of the microbead. The embolic microbead according to an exemplary embodiment of the present disclosure may include the biocompatible polymer in the amount of about 97 parts or more by weight based on 100 parts by weight of the microbead. The embolic microbead according to an exemplary embodiment of the present disclosure may include the biocompatible polymer in the amount of about 99 parts or more by weight based on 100 parts by weight of the microbead.

The biocompatible polymer includes gelatin cross-linked with an iron adsorption block (iron adsorption polymer). The iron adsorption block (iron adsorption polymer) may function to adsorb iron inside the biocompatible polymer.

The iron adsorption block includes a fucoidan and/or a polyphenol. Further, the iron adsorption block may include the fucoidan, or the fucoidan and polyphenol.

The iron (Fe) adsorption block may have a high concentration of hydroxyl group (-OH). Therefore, the iron adsorption block can adsorb a large amount of iron ions.

The polyphenol is an aromatic alcohol compound extracted from plants. The polyphenol is a functional group having one molecule with two or more phenol groups, and may have several hydroxyl groups. The polyphenol may be a biodegradable anionic polyphenol, and the polyphenol may include at least one selected from tannin, gallic acid, tannic acid, catechin, epicatechin, epigallocatechin gallate, quercetin, resveratrol, isoflavone, flavonoids, and polyphenols derived from seaweeds.

Also, the polyphenol may include catechins extracted from green tea, resveratrols extracted from wine or quercetins extracted from apples or onions. Further, the polyphenol may include flavonoids extracted from fruits or isoflavones extracted from soybeans.

The polyphenol may include epigallocatechin gallate (EGCG).

The embolic microbead according to an exemplary embodiment of the present disclosure includes the iron adsorption block and thus can effectively adsorb iron ions.

The gelatin may be aminated gelatin, and the embolic microbead may include cross-linking of a cationic aminated gelatin and an anionic fucodian. The gelatin may include an amine group and a carboxylic acid group. Here, the aminated gelatin may have a relatively high concentration of the amine group. More specifically, the aminated gelatin may have an improved concentration of an amine group by reacting normally available gelatin with amine. Examples of the amine may include ethylenediamine (1,2-diaminoethane), 1,1-dimethylethylenediamine, 1,2-dimethylethylenediamine, ethambutol, tetrakis(dimethylamino)ethylene or tetramethylethylenediamine (TMEDA).

A weight ratio of the gelatin : the iron adsorption block may be about 4:1 to about 10:1, but may not be limited thereto. The weight ratio of the gelatin : the iron adsorption block may be about 4:1 to about 10:1, about 4:1 to about 9:1, about 4:1 to about 8:1, about 4:1 to about 7:1, about 4:1 to about 6:1, about 4:1 to about 5:1, about 5:1 to about 10:1, about 5:1 to about 9:1, about 5:1 to about 8:1, about 5:1 to about 7:1, about 5:1 to about 6:1, about 6:1 to about 10:1, about 6:1 to about 9:1, about 6:1 to about 8:1, about 6:1 to about 7:1, about 7:1 to about 10:1, about 7:1 to about 9:1, about 7:1 to about 8:1, about 8:1 to about 10:1, about 8:1 to about 9:1, or about 9:1 to about 10:1. The weight ratio of the gelatin : the iron adsorption block may be about 5:1 to about 7:1.

Also, the biocompatible polymer includes gelatin. The biocompatible polymer may include gelatin having different molecular weights. The biocompatible polymer may include a first gelatin having a first average molecular weight; and a second gelatin having a second average molecular weight smaller than the first average molecular weight. Further, the biocompatible polymer may further include a third gelatin having a third average molecular weight which is smaller than the second average molecular weight, and the first gelatin, the second gelatin and the third gelatin may be cross-linked to one another. Further, the biocompatible polymer may further include a fourth gelatin having a fourth average molecular weight which is smaller than the third average molecular weight, and the first gelatin, the second gelatin, the third gelatin and the fourth gelatin may be cross-linked to one another.

The first average molecular weight may be 40,000 to 100,000, 15,000 to 39,000 or 5,000 to 14,000, and the second average molecular weight may be 15,000 to 39,000, 5,000 to 14,000 or 500 to 4,000.

The first average molecular weight may be 40,000 to 100,000 or 15,000 to 39,000, the second average molecular weight may be 15,000 to 39,000 or 5,000 to 14,000, and the third average molecular weight may be 5,000 to 14,000 or 500 to 4,000.

Further, the first average molecular weight may be 40,000 to 100,000, the second average molecular weight may be 15,000 to 39,000, the third average molecular weight may be 5,000 to 14,000, and the fourth average molecular weight may be 500 to 4,000. The first average molecular weight may be 40,000 to 60,000, the second average molecular weight may be 15,000 to 30,000, the third average molecular weight may be 7,000 to 12,000, and the fourth average molecular weight may be 2,000 to 5,000.

The gelatin may include a greater amount of gelatin having a relatively high average molecular weight than gelatin having a relatively low average molecular weight. In this case, the embolic microbead formed by cross-linking the gelatin is relatively slowly degraded in vivo.

The gelatin may include a greater amount of gelatin having a relatively low average molecular weight than

gelatin having a relatively high average molecular weight. In this case, the embolic microbead formed by cross-linking the gelatin is relatively fast degraded in vivo.

If the gelatin included in the biocompatible polymer include the first gelatin having the first average molecular weight and the second gelatin having the second average molecular weight which is smaller than the first average molecular weight, the biocompatible polymer may include the first gelatin in the amount of about 80 parts by weight or less, about 75 parts by weight or less, about 70 parts by weight or less, about 65 parts by weight or less or about 60 parts by weight or less based on 100 parts by weight of the gelatin.

The average molecular weight described above or to be described below refers to the weight average molecular weight. Also, the average molecular weight may have a unit of Dalton.

The average molecular weights of the first gelatin, the second gelatin, the third gelatin and the fourth gelatin may be regulated through a hydrolysis process. That is, gelatin having a high average molecular weight may be hydrolyzed and gelatin having a low average molecular weight may be produced.

The gelatin may include mammal-derived gelatin or fish-derived gelatin. In particular, if the gelatin includes the fish-derived gelatin, the risk of mad cow disease or foot-and-mouth disease of conventional gelatin derived from cows or pigs can be eliminated.

If the gelatin is composed of the fish-derived gelatin, the embolic microbead according to an exemplary embodiment of the present disclosure may be formed at a relatively low temperature and may have a uniform porous spherical shape.

The gelatin may include glycine, proline and hydroxyproline. Therefore, the embolic microbead according to an exemplary embodiment of the present disclosure may have elasticity, flexibility and expansibility depending on reaction conditions. Therefore, the embolic microbead according to an exemplary embodiment of the present disclosure may be applied to blood vessels so that the blood blocking time can be predicted.

The gelatin may be fish-derived gelatin. The fish-derived gelatin is prepared from skins of warm current fish and may be gelated at a lower temperature than gelatin derived from cows or pigs. For example, the fish-derived gelatin may be easily cross-linked in a sol state at a temperature of from about 20°C to about 40°C, and the embolic microbead according to an an exemplary embodiment of the present disclosure may be prepared.

Also, the gelatin may be prepared from animal-derived collagen or fish-derived collagen. The gelatin may be extracted from the collagen when heat is applied to the collagen. Further, as the gelatin, marine collagen peptide or collagen peptide is available from the market.

The fucoidan may be extracted from brown algae such as sea mustard, mozuku cladosiphon novae-caledoniae kylin), kelp and hizikia fusiforme. Also, the fucoidan may include a sulfuric acid group and a polysaccharide such as fucose. That is, the fucoidan may have a chemical structure in which the sulfuric acid group and the polysaccharide are coupled to each other.

The fucoidan may include glucuronic acid represented by the following Chemical Formula 1.

[Chemical Formula 1]

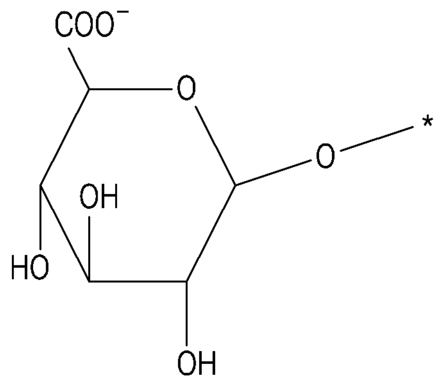

The fucoidan may include a unit represented by the following Chemical Formula 2.

[Chemical Formula 2]

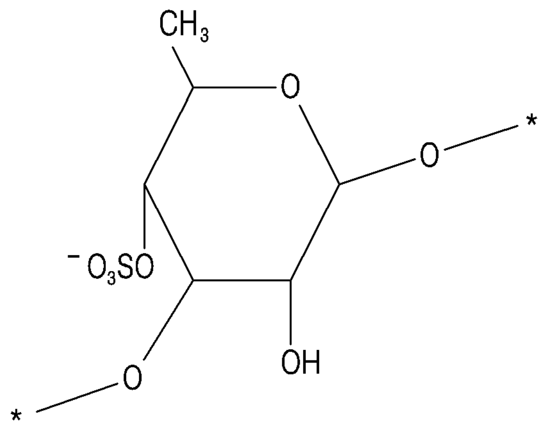

The fucoidan may have an average molecular weight of from about 3,000 to about 40,000. The fucoidan may have the average molecular weight of from about 5,000 to about 30,000. The fucoidan may have the average molecular weight of from about 6,000 to about 25,000. The fucoidan may have an average molecular weight of from about 7,000 to about 20,000.

Since the fucoidan simultaneously includes the hydroxyl group and the sulfuric acid group, it can adsorb iron ions and can be efficiently coupled to an anticancer agent such as doxorubicin or the like. Therefore, the embolic microbead according to an exemplary embodiment of the present disclosure can adsorb iron ions and effectively deliver an anticancer agent such as doxorubicin or the like.

Also, the fucoidan may help improve the liver function. That is, the fucoidan can be used as an adjuvant for improving the liver function. Further, the fucoidan may be used as an adjuvant having an anticancer effect. Therefore, the embolic microbead according to an exemplary embodiment of the present disclosure can be effective in liver cancer or the like.

The embolic microbead according to an exemplary embodiment of the present disclosure may have a high iron adsorption rate. The embolic microbead according to an exemplary embodiment of the present disclosure may have a high iron adsorption rate as shown in the following Equations 1 to 5.

In an exemplary embodiment of the present disclosure, an iron adsorption rate after 24 hours(IAd24) based on 100 mg of the embolic microbead, represented by the following Formula 1, may be about 5 %/100 mg or more:

[Formula 1] $$\mathrm{IAd24} = \frac{(IC0-IC24)}{IC0} \times 100$$ ;

in the Formula 1, the IC0 is an initial molar concentration of a $FeCl_3$ aqueous solution, and the IC24 is a molar concentration of an iron ion of the $FeCl_3$ aqueous solution left for 24 hours after adding the embolic microbead to the $FeCl_3$ aqueous solution.

The iron adsorption rate after 24 hours(IAd24) may be about 6 %/100 mg or more. The iron adsorption rate after 24 hours(IAd24) may be about 7 %/100 mg or more. The iron adsorption rate after 24 hours(IAd24) may be about 8 %/100 mg or more. The iron adsorption rate after 24 hours(IAd24) may be about 9 %/100 mg or more. The iron adsorption rate after 24 hours(IAd24) may be about 10 %/100 mg or more. The iron adsorption rate after 24 hours(IAd24) may be about 50 %/100 mg or less.

In an exemplary embodiment of the present disclosure, an iron adsorption rate after 48 hours(IAd48) based on 100 mg of the embolic microbead, represented by the following Formula 2, may be about 5 %/100 mg or more:

[Formula 2] $$\mathrm{IAd48} = \frac{(IC0 - IC48)}{IC0} \times 100$$ ;

in the Formula 2, the IC0 is an initial molar concentration of a $FeCl_3$ aqueous solution, and the IC48 is a molar concentration of an iron ion of the $FeCl_3$ aqueous solution left for 48 hours after adding the embolic microbead to the $FeCl_3$ aqueous

solution.

The iron adsorption rate after 48 hours(IAd48) may be about 6 %/100 mg or more. The iron adsorption rate after 48 hours(IAd48) may be about 7 %/100 mg or more. The iron adsorption rate after 48 hours(IAd48) may be about 8 %/100 mg or more. The iron adsorption rate after 48 hours(IAd48) may be about 9 %/100 mg or more. The iron adsorption rate after 48 hours(IAd48) may be about 10 %/100 mg or more. The iron adsorption rate after 48 hours(IAd48) may be about 50 %/100 mg or less.

In an exemplary embodiment of the present disclosure, an iron adsorption rate after 1 hour(IAd1) based on 100 mg of the embolic microbead, represented by the following Formula 3, may be about 5 %/100 mg or more:

[Formula 3]
$$\mathrm{IAd1} = \frac{(IC0 - IC1)}{IC0} \times 100$$
;

in the Formula 3, the IC0 is an initial molar concentration of a $FeCl_3$ aqueous solution, and the IC1 is a molar concentration of an iron ion of the $FeCl_3$ aqueous solution left for 1 hour after adding the embolic microbead to the $FeCl_3$ aqueous solution.

The iron adsorption rate after 1 hour(IAd1) may be about 6 %/100 mg or more. The iron adsorption rate after 1 hour(IAd1) may be about 7 %/100 mg or more. The iron adsorption rate after 1 hour(IAd1) may be about 8 %/100 mg or more. The iron adsorption rate after 1 hour(IAd1) may be about 9 %/100 mg or more. The iron adsorption rate after 1 hour(IAd1) may be about 10 %/100 mg or more. The iron adsorption rate after 1 hour(IAd1) may be about 50 %/100 mg or less.

In an exemplary embodiment of the present disclosure, an iron adsorption rate after 12 hours(IAd12) based on 100 mg of the embolic microbead, represented by the following Formula 4, may be about 5 %/100 mg or more:

[Formula 4]
$$\mathrm{IAd12} = \frac{(IC0 - IC12)}{IC0} \times 100$$
;

in the Formula 4, the IC0 is an initial molar concentration of a $FeCl_3$ aqueous solution, and the IC12 is a molar concentration of an iron ion of the $FeCl_3$ aqueous solution left for 12 hours after adding the embolic microbead to the $FeCl_3$ aqueous solution.

The iron adsorption rate after 12 hours(IAd12) may be about 6 %/100 mg or more. The iron adsorption rate after 12 hours(IAd12) may be about 7 %/100 mg or more. The iron adsorption rate after 12 hours(IAd12) may be about 8 %/100 mg or more. The iron adsorption rate after 12 hours(IAd12) may be about 9 %/100 mg or more. The iron adsorption rate after 12 hours(IAd12) may be about 10 %/100 mg or more. The iron adsorption rate after 12 hours(IAd12) may be about 50 %/100 mg or less.

In an exemplary embodiment of the present disclosure, an iron adsorption rate after 72 hours(IAd72) based on 100 mg of the embolic microbead, represented by the following Formula 5, may be about 5 %/100 mg or more:

[Formula 5]
$$\mathrm{IAd72} = \frac{(IC0 - IC72)}{IC0} \times 100$$
;

in the Formula 5, the IC0 is an initial molar concentration of a $FeCl_3$ aqueous solution, and the IC72 is a molar concentration of an iron ion of the $FeCl_3$ aqueous solution left for 72 hours after adding the embolic microbead to the $FeCl_3$ aqueous solution.

The iron adsorption rate after 72 hours(IAd72) may be about 6 %/100 mg or more. The iron adsorption rate after 72 hours(IAd72) may be about 7 %/100 mg or more. The iron adsorption rate after 72 hours(IAd72) may be about 8 %/100 mg or more. The iron adsorption rate after 72 hours(IAd72) may be about 9 %/100 mg or more. The iron adsorption rate after 72 hours(IAd72) may be about 10 %/100 mg or more. The iron adsorption rate after 72 hours(IAd72) may be about 50 %/100 mg or less.

In the Formula 1 to 5, the IC0 may be 0.18 M.

The embolic microbead according to an exemplary embodiment of the present disclosure may include a biocompatible polymer, the biocompatible polymer includes a gelatin cross-linked with an iron adsorption block, and the

embolic microbead may be combined with an anticancer drug. Herein, the embolic microbead may chelate an anticancer agent.

The coupled anticancer agent is not specifically limited, and any anticancer agent which can be coupled to the embolic microbead according to an exemplary embodiment of the present disclosurecan be used without limitations. For example, the the anticancer drug may be selected from doxorubicin, irinotecan, gemcitabine, oxaliplatin and docetaxel.

The embolic microbead according to an exemplary embodiment of the present disclosure may have a high anticancer conjugation rate, particularly a high doxorubicin conjugation rate. In particular, since the biocompatible polymer has the fucoidan, the embolic microbead according to an exemplary embodiment of the present disclosure may have a high iron adsorption rate as well as a high anticancer conjugation rate, particularly a high doxorubicin conjugation rate.

In an exemplary embodiment of the present disclosure, a combining rate with the anticancer drug after 1 hour(AAd) based on 100 mg of the embolic microbead, represented by the following Formula 6, may be about 10 %/100 mg or more:

[Formula 6] $$AAd = \frac{(AC0-AC1)}{AC0} \times 100$$ ;

in the Formula 6, the AC0 is an initial mg/mℓ concentration of an anticancer drug solution, and the AC1 is a mg/mℓ concentration of the anticancer drug of the anticancer drug solution left for 1 hour after adding the embolic microbead to the anticancer drug solution. Herein, the anticancer drug may be doxorubicin.The combining rate(AAd) may be about 10 %/100 mg or more. The combining rate(AAd) may be about 20 %/100 mg or more. The combining rate(AAd) may be about 30 %/100 mg or more. The combining rate(AAd) may be about 50 %/100 mg or more. The combining rate(AAd) may be about 60 %/100 mg or more. The combining rate(AAd) may be about 90 %/100 mg or less.

Herein, the AC0 may be about 1 mg/mℓ.

The embolic microbead according to an exemplary embodiment of the present disclosure is formed by cross-linking the gelatin and the iron adsorption block. The embolic microbead according to an exemplary embodiment of the present disclosure includes a cross-linker. The cross-linker serves to cross-link the gelatin and the iron adsorption block.

Here, the cross-linker includes at least one selected from the group consisting of formaldehyde, glutaraldehyde, dialdehyde starch, epoxy compound, glutaraldehyde glyoxal, glyoxal, 2 2-dimethoxy-2-phenylacetophenone, tripolyphosphate sodium salt, diacetaldehyde PEG, scleraldehyde, diethyl squarate, epichlorohydrin and genipin.

The embolic microbead according to an exemplary embodiment of the present disclosure may include the cross-linker in the amount of from about 0.20 parts by weight to about 50 parts by weight based on 100 parts by weight of the gelatin. More specifically, the embolic microbead may may include the cross-linker in the amount of from about 3 parts by weight to about 20 parts by weight based on 100 parts by weight of the gelatin.

The embolic microbead according to an exemplary embodiment of the present disclosure may have an average particle size of from about 10 μm to about 2000 μm. More specifically, the embolic microbead according to an exemplary embodiment of the present disclosure may have an average particle size of from about 50 μm to about 2000 μm. More specifically, the embolic microbead according to an exemplary embodiment of the present disclosure may have an average particle size of from about 50 μm to about 1000 μm.

The embolic microbead according to an exemplary embodiment of the present disclosure has a porous structure. The embolic microbead according to an exemplary embodiment of the present disclosure may have a sponge structure. That is, the embolic microbead according to an exemplary embodiment of the present disclosure may include micropores. The embolic microbead according to an exemplary embodiment of the present disclosure may have a porosity of from about 20 vol% to about 70 vol%. Therefore, the embolic microbead according to an exemplary embodiment of the present disclosure may have a high elasticity and a high swelling coefficient.

The embolic microbead according to an exemplary embodiment of the present disclosure is formed by cross-linking gelatin having a relatively high average molecular weight and gelatin having a relatively low average molecular weight. Therefore, the embolic microbead according to an exemplary embodiment of the present disclosure may have a desired particle size and a desired degradation rate. For example, an average diameter of the embolic microbead according to an exemplary embodiment of the present disclosure may be determined by the amount and the average molecular weight of the gelatin having a relatively high average molecular weight. Also, a degradation rate of the embolic microbead according to an exemplary embodiment of the present disclosure may be determined by the amount and the average molecular weight of the gelatin having a relatively low average molecular weight.

Therefore, the embolic microbead according to an exemplary embodiment of the present disclosure may have a desired particle size and a desired degradation rate depending on the amounts and average molecular weights of the

first gelatin and the second gelatin. As a result, the embolic microbead according to an exemplary embodiment of the present disclosure may have an appropriate size and an appropriate degradation rate, and when it is embolized into a target blood vessel, it can maintain an embolic effect until a desired time and then can be degraded.

Particularly, the embolic microbead according to an exemplary embodiment of the present disclosure may have a high degradation rate, and after it is injected into a patient, it can be replaced and injected into other blood vessels of the patient many times depending on the conditions of the patient. Therefore, the embolic microbead according to an exemplary embodiment of the present disclosure can effectively treat the patient.

The embolic microbead according to an exemplary embodiment of the present disclosure is prepared using gelatin. Therefore, the embolic microbead according to an exemplary embodiment of the present disclosure is excellent in biocompatibility, expansibility, elasticity and flexibility. Also, the embolic microbead according to an exemplary embodiment of the present disclosure may have various particle sizes of from about 50 μm to about 2000 μm, and the embolic microbead according to an exemplary embodiment of the present disclosure has a porous spherical shape having a uniform sponge structure. Therefore, the embolic microbead according to an exemplary embodiment of the present disclosure can minimize blood vessel stimulation.

Also, the embolic microbead according to an exemplary embodiment of the present disclosure may be prepared by cross-linking various amounts of gelatin having various average molecular weights. Therefore, the embolic microbead according to an exemplary embodiment of the present disclosure may be used alone or in combination so that the blood vessel blocking time can be predicted.

Also, the embolic microbead according to an exemplary embodiment of the present disclosure has the high iron adsorption rate after 24 hours(IAd24) and the high iron adsorption rate after 48 hours(IAd48). Therefore, the embolic microbead according to an exemplary embodiment of the present disclosure is capable of fast iron adsorption.

Therefore, the embolic microbead according to an exemplary embodiment of the present disclosureis injected into a human body, particularly the liver carotid artery so that blood flow to cancer cells or the like can be blocked and the supply of iron ions, which are required for existence or proliferation of cancer cells, to the cancer cells can be blocked.

Therefore, the embolic microbead according to an exemplary embodiment of the present disclosure can effectively necrotize cancer cells by blocking a blood vessel.

Also, the embolic microbead according to an exemplary embodiment of the present disclosure can effectively adsorb iron ions that are eluted when cancer cells or the like are necrotized.

Therefore, the embolic microbead according to an exemplary embodiment of the present disclosure can effectively suppress regrowth of cancer cells.

The embolic microbead according to an exemplary embodiment of the present disclosure can be very effectively used for treating cancers, such as liver cancer, and treating proliferative diseases.

Referring to **FIG. 1**, the embolic microbead according to an example may be prepared by the following process.

First, gelatin is prepared (S10). The gelatin maybe mammal-derived gelatin or fish-derived gelatin. The gelatin may have a weight average molecular weight of from about 50,000 to about 100,000.

The gelatin may be obtained from collagen of mammals or collagen of fish. Otherwise, the gelatin may be commercially obtained. The gelatin can be referred to the above description.

Then, the gelatin may be hydrolyzed to have an appropriate molecular weight. The gelatin may be hydrolyzed in a subcritical water state.

More specifically, the gelatin is dissolved or dispersed in water to form an aqueous solution or aqueous dispersion. Here, the amount of the gelatin in the aqueous solution or aqueous dispersion may be from about 3 wt% to about 20 wt%.

Then, the aqueous solution or aqueous dispersion is maintained in the subcritical water state for a predetermined period of time. For example, the aqueous solution or aqueous dispersion may be maintained at a temperature of from about 100°C to about 250°C and a pressure of from about 30 atm. to about 80 atm for from about 10 minutes to about 5 hours. Therefore, the gelatin is hydrolyzed to form a polypeptide. Also, the hydrolysis process in the subcritical water state may be performed in the presence of carbon dioxide ($CO_2$) and nitrogen ($N_2$).

Further, the gelatin may be hydrolyzed with an enzyme. Furthermore, the gelatin may be hydrolyzed with an acid. The gelatin may be hydrolyzed with both the subcritical water and the enzyme.

Then, the hydrolyzed gelatin may be fractionated. The hydrolyzed gelatin may be fractionated by molecular weight in water serving as a solvent by gel filtration chromatography. The hydrolyzed gelatin may be fractionated by an HPLC (1260 HPLC system, Agilent, USA) connected to a fraction collector (G1364C 1260 FC-AS, Agilent, USA) and divided by molecular weight. A polypeptide divided by molecular weight may be obtained by various methods capable of fractionating an oligomer or polymer by molecular weight. The hydrolyzed gelatin may be separated by molecular weight through a column.

Therefore, gelatin with different molecular weights can be obtained. For example, the gelatin, the second gelatin and the third gelatin may be obtained.

As described above, gelatin divided by molecular weight may be obtained.

For example, the hydrolyzed gelatin is fractionated so that gelatin having an average molecular weight of from about 40,000 to about 60,000, gelatin having an average molecular weight of from about 20,000 to about 30,000, gelatin having an average molecular weight of from about 8,000 to about 12,000 and gelatin having an average molecular weight of from about 1,000 to about 2,000 may be obtained.

Then, the gelatin may be aminated. The aminated gelatin may be effectively reacted with the iron adsorption polymer, particularly the fucoidan and/or polyphenol.

The amination process may be performed as follows.

The gelatin and a sufficient amount of diamine are mixed under subacid conditions, for example, at a pH of from about 4.5 to about 6. Then, a catalyst is added into the mixed solution of the gelatin and the diamine and then reacted at a temperature of from about 30°C to about 45°C. Thus, the aminated gelatin may be prepared.

Thereafter, the iron adsorption polymer is prepared (S20). The fucoidan may be extracted and prepared from algae. Also, the polyphenol may be extracted and prepared from plants such as green tea.

Then, the gelatin and the iron adsorption polymer are cross-linked by a cross-linker (S30).

The cross-linker may be the same as described above

The cross-link reaction is carried out in an aqueous solution that includes the gelatin and the iron adsorption polymer in the amount of form about 3 wt% to about 10 wt%. After the cross-linker is added into the aqueous solution in the amount as described above, the gelatin and the iron adsorption polymer are cross-linked by the cross-linker at a temperature of from about 20°C to about 40°C for from about 6 hours to about 12 hours.

For example, the aqueous solution of the gelatin and the iron adsorption polymer and the cross-linker solution are mixed at a weight ratio of from about 100:0.5 to about 100:10. Then, the mixed solution is firstly stirred at a temperature of from about 20°C to about 40°C and a speed of from about 1000 rpm to about 1500 rpm for from about 5 minutes to about 120 minutes. Thereafter, the firstly stirred mixed solution is secondly stirred at a temperature of from about 20°C to about 40°C and a speed of from about 1 rpm to about 500 rpm for from about 10 minutes to about 48 hours. Therefore, a polymer aqueous solution in which the gelatin and the iron adsorption polymer are cross-linked can be prepared.

More specifically, in the first stirring process, if the stirring speed exceeds the range of stirring speed of from about 1000 rpm to about 1500 rpm, bubbles are formed, which may cause a decrease in density. Also, in the first stirring process, if the stirring is performed at a temperature of about 20°C or less, the mixed solution may be gelated. Further, in the first stirring process, if the stirring is performed at a temperature of about 40°C or more, bubbles are formed, which may cause a decrease in density.

Further, in the second stirring process, if the stirring speed exceeds about 500 rpm, the mixed solution may be gelated and thus cannot be easily cross-linked to a sol state, which may cause a remarkable decrease in absorbency. Therefore, if the stirring speed exceeds about 500 rpm, the function of the embolic microbead may deteriorate.

Furthermore, in the second stirring process, if the temperature exceeds the above-described range of temperature, the mixed solution may be gelated and hardened, which may make it difficult to prepare a cross-linked gelatin aqueous solution. In the second stirring process, the stirring is performed for from about 10 minutes to about 48 hours in order to improve the absorbency and maintain the shape.

Therefore, a cross-linked gelatin aqueous solution is prepared through the stirring process. Then, the cross-linked gelatin aqueous solution is dripped or dispersed onto oil so that aggregation of the cross-linked gelatin in a gel state can be suppressed. Also, a porous spherical cross-linked gelatin particle having a uniform sponge structure with a particle size of from about 50 μm to about 2,000 μm can be prepared. Further, the cross-linked gelatin is less exposed to the oil, the oil can be easily separated from the cross-linked gelatin.

A cross-linked gelatin particle having a spherical sponge structure is formed. The cross-linked gelatin particle may have various average particle sizes depending on the amounts of the gelatin having various average molecular weights, the amount of the fucoidan or polyphenol, the amount of the cross-linker, the cross-linking temperature and the cross-linking time. For example, the cross-linked gelatin particle may have an average particle size of from about 50 μm to about 2000 μm under the above-described conditions.

More specifically, the gelatin may be fish gelatin. That is, gelatin used for the hydrolysis may be derived from fish. Particularly, the fish gelatin is prepared from skins of warm current fish and may be gelated at a lower temperature than gelatin derived from cows or pigs. Therefore, the fish gelatin may be easily cross-linked in a sol state at a temperature of from about 20°C to about 40°C to form a porous sphere having a uniform sponge structure with a particle size of from about 50 μm to about 2000 μm. If the gelatin is derived from the fish gelatin, it has a low viscosity in a non-gel state and has a low molecular weight. Thus, it can be dripped or dispersed at a higher concentration. Therefore, in the present example, gelatin microparticles having a high apparent density can be prepared.

Also, the concentration of the gelatin derived from the fish gelatin in an aqueous solution may be from about 3 wt% to about 20 wt%. Further, in the second stirring process, the concentration of the cross-linker in the prepared cross-linked gelatin aqueous solution may be from about 0.05 wt% to about 10 wt%.

More specifically, an emulsion of the gelatin particles is allowed to pass through a nozzle with compressed air in an encapsulator and dispersed or dripped onto oil. Here, the particle solution may have a temperature of from about

20°C to about 40°C and may pass through the nozzle at a temperature of from about 65°C to about 75°C. Further, by setting a vibration frequency in a range of from about 40 Hz to about 6000 Hz, it is possible to control the size of a spherical gelatin liquid droplet to be dripped or dispersed.

Furthermore, the oil may be ester-based oil, hydrocarbon-based oil, animal oil or vegetable oi. Specifically, the oil may be olive oil, bean oil, canola oil, grapeseed oi, soybean oil, palm oil, paraffin oil, α-bisabolol, stearyl glycyrrhetinate, salicylic acid, tocopheryl acetate, panthenol, glyceryl stearate, cetyl octanolate, isopropyl myristate, 2-ethylene isopelagonate, di-c12-13 alkyl malate, cetearyl octanoate, butylene glycol dicaprylate/dicaprate, isononyl isostearate, isostearyl isostearate, cetyl octanoate, octyldodecyl myristate, cetyl esters, c10-30 cholesterol/lanosterol ester, hydrogenated castor oil, mono-glycerides, diglycerides, triglycerides, beeswax, canauba wax, sucrose distearate, PEG-8 beeswax, candelilla(euphorbia cerifera) wax, mineral oil, squalene, squalane, monoglycerides, middle chain glycerides, myglyol or cremophor.

Desirably, the oil may be paraffin oil, but is not limited thereto. More desirably, the oil may be mixed with a cross-linker in the amount of from about 0 wt% to about 5 wt% based on 100 wt% of the oil in order for the gelatin particles to easily maintain their spherical shape on the oil.

Further, the oil is cooled and maintained at a temperature of from about 0°C to about 10°C, which makes it easy to form spherical gelatin.

Therefore, a cross-linked gelatin particle can be prepared.

Then, the cross-linked gelatin particle is washed and dried (S40). After the oil including the spherical gelatin particles is stirred, washing and drying processes are performed. Thus, a porous gelatin microparticle having a sponge structure can be formed.

The cross-linked spherical gelatin particle may be washed with an organic solvent.

The oil including the spherical gelatin particles prepared in the process S40 is stirred at a temperature of from about 0°C to about 10°C and a speed of from about 400 rpm to about 1400 rpm, settled and then washed with an organic solvent. Therefore, the oil and the cross-linker included in the cross-linked spherical gelatin particle are removed, and the cross-linked spherical gelatin particle is solidified.

Then, the solidified gelatin particle is lyophilized for from about 3 hours to about 48 hours. Therefore, a porous gelatin microparticle having a sponge structure is formed. Here, the porous gelatin microparticle having a sponge structure has a particle size of from about 150 μm to about 2000 μm.

More specifically, in the washing and drying processes, the oil including the gelatin particles is stirred at a speed of from about 400 rpm to about 1400 rpm, and, thus, a spherical gelatin particle can be easily formed and the spherical gelatin can have appropriate properties. Also, since the oil is maintained in the range of temperature of from about 0°C to about 10°C, aggregation of the spherical gelatin particles on the oil can be suppressed and the spherical shape can be maintained.

Further, the organic solvent is petroleum ether, ethyl ether, isopropyl acetate, n-propyl acetate, isobutyl acetate, n-butyl acetate, isobutyl isobutyrate, 2-ethylhexyl acetate, ethylene glycol diacetate, C9 acetate, C10 acetate, methyl ethyl ketone, methyl isobutyl ketone, methyl isoamyl ketone, methyl n-amyl ketone, dibutyl ketone, cyclohexanone, isophorone, acetaldehyde, n-butyl aldehyde, croton aldehyde, 2-ethyl hexaldehyde, isobutyl aldehyde, propion aldehyde, ethyl 3-ethoxypropionate, toluene, xylene, trichloroethane, propylene glycol monomethyl ethyl acetate, ethylene glycol monoethyl ether acetate, ethylene glycol monobutyl ether, diethylene glycol monobutyl ether acetate, dibutyl phthalate, diethyl phthalate, dimethyl phthalate, dioctyl phthalate, dioctyl terephthalate, butyl octyl phthalate, butyl benzene phthalate, dioctyl adipate, triethylene glycol di-2-ethylhexanoate, trioctyl trimethylate, glyceryl triacetate, glyceryl/tripropionin, 2,2,4-trimethyl-1,3-pentanediol diisobutyrate or a mixture thereof.

Desirably, the organic solvent is ethyl ether or isobutyl acetate.

Then, the embolic microbead according to an exemplary embodiment of the present disclosure is added into deionized water to prepare a formulation (S50).

As described above, the embolic microparticle according to the present disclosure is prepared form fish gelatin and thus is excellent in biocompatibility, expansibility, elasticity and flexibility. The embolic microparticle according to the present disclosure is formed into a porous sphere having a uniform sponge structure with a particle size of from about 150 μm to about 2000 μm, which minimizes blood vessel stimulation and makes it easy to block a blood vessel. The embolic microparticle according to the present disclosure is prepared by the following preparation method.

Also, since the embolic microbead according to an exemplary embodiment of the present disclosure is formed by cross-linking the gelatin having different molecular weights, the embolic microbead according to an exemplary embodiment of the present disclosure can be degraded in vivo at a desired time point.

Further, the embolic microbead according to the present disclosure includes the fucoidan and/or the polyphenol as the iron adsorption polymer and thus has an improved iron adsorption rate.

Therefore, the embolic microbead according to an exemplary embodiment of the present disclosure can effectively remove cancer cells.

A composition for treating proliferative diseases according to an exemplary embodiment of the present disclosure

may include an embolic microbead, the embolic microbead includes gelatin crosslinked with an iron adsorption block, and the embolic microbead may be combined with an iron component and/or an anticancer drug. The embolic microbead may adsorb or may be coupled to or may chelate an iron component and/or an anticancer agent.

The proliferative diseases may include at least one selected from neovascularization, tumor, cancer, bone diseases, fibroplasia disorders, statomegaly, renal cyst, and atherosclerosis.

The cancer may include at least one selected from solid tumor, hematologic malignancy, colorectal cancer, uterine cancer, uterine myoma, meningioma, lung cancer, small cell lung cancer, non-small cell lung cancer, gastrointestinal cancer, bowel cancer, breast cancer, ovarian cancer, prostate cancer, testicular cancer, liver cancer, renal cancer, bladder cancer, pancreatic cancer, sarcoma, osteosarcoma, kaposi's sarcoma and melanoma. Particularly, the cancer may be the liver cancer.

**MODE FOR CARRYING OUT THE INVENTION**

Hereinafter, the present disclosure will be explained in more detail with reference to Examples. However, the following Examples are illustrative only for better understanding of the present disclosure but do not limit the present disclosure.

**Preparation Example**

**Preparation of Aminated Gelatin**

A first gelatin solution was prepared by mixing 10 g of first gelatin (with an average molecular weight of about 42,000) in 250 mL of 0.1 M phosphate buffer solution (pH 5.3). After a 1,2-ethylenediamine solution was added into 5.6 g of the first gelatin solution, the pH was adjusted to 5 using HCl. Then, 5.35 g of 1-ethyl-3-(3-dimethylaminopropyl)carbodiimide hydrochloride was added as a catalyst into the first gelatin solution. Thereafter, the first gelatin solution added with the 1,2-ethylenediamine solution and the catalyst was reacted at a temperature of 37°C for about 8 hours. Then, the reacted first gelatin solution was washed with deionized water through dialysis for 48 hours, and first aminated gelatin was prepared through lyophilization.

A second gelatin solution was prepared by mixing 10 g of second gelatin (with an average molecular weight of about 31,000) in 250 mL of 0.1 M phosphate buffer solution (pH 5.3). After a 1,2-ethylenediamine solution was added into 5.6 g of the second gelatin solution, the pH was adjusted to 5 using HCl. Then, 1-ethyl-3-(3-dimethylaminopropyl)carbodiimide hydrochloride was added as a catalyst into the second gelatin solution. Thereafter, the second gelatin solution added with the 1,2-ethylenediamine solution and the catalyst was reacted at a temperature of 37°C for about 8 hours. Then, the reacted second gelatin solution was washed with deionized water through dialysis for 48 hours, and second aminated gelatin was prepared through lyophilization.

**Example 1.**

**Preparation of Cross-linked Gelatin Particle**

A gelatin aqueous solution of about 5 wt% was prepared by dissolving 35 parts by weight of the first aminated gelatin, 15 parts by weight of the second aminated gelatin and 7.5 parts by weight of fucoidan (MSC, kelp fucoidan material) in deionized water. Then, a cross-linker aqueous solution was prepared by adding 37 mL of formaldehyde in 100 mL of an aqueous solution containing 15 wt% methanol.

Then, an aqueous solution in which 0.2 mL of the cross-linker aqueous solution was added to and mixed with 20 mL of the gelatin aqueous solution was firstly stirred at a temperature of about 30°C and a speed of 1000 rpm for 30 minutes and then secondly stirred at a temperature of 30°C and a speed of 200 rpm for 24 hours to prepare a cross-linked gelatin aqueous solution in a sol state.

Thereafter, the prepared cross-linked gelatin aqueous solution was injected into an Encapsulator B-390 (BUCHI, Switzerland) whose vibration frequency is adjusted to 400 Hz, whose electronegativity is adjusted to 1200 V and whose nozzle temperature is adjusted to 75°C and then dripped onto 100 mL of paraffin oil containing a cross-linker by using compressed air in the Encapsulator B-390. Thus, a spherical gelatin particle was formed on the paraffin oil. The paraffin oil used herein contains 1 mL of 37% formaldehyde in 100 mL of paraffin oil.

Then, the paraffin oil including the spherical gelatin particle was stirred at 0°C and 1000 rpm and then settled for 30 minutes.

Thereafter, the spherical gelatin droplet was washed by adding isopropyl ethyl alcohol thereto so that the spherical gelatin was solidified, and the paraffin oil and the formaldehyde were removed.

The solidified gelatin spheres were lyophilized for 24 hours to prepare a gelatin microbead including fucoidan.

The gelatin microbead was used alone or mixed with deionized water at a ratio of 5:5.

**Examples 2 to 8 and Comparative Examples 1 and 2**

The gelatin microbead was prepared in the same manner as in Example 1 except the amounts of the first aminated gelatin, the second aminated gelatin, the fucoidan and epigallocatechin gallate (EGCG, produced by R&O) shown in the following Table 1.

However, in Examples 5 to 8, EGCG was added to the gelatin aqueous solution according to the parts by weight shown in the following Table 1. Also, in Comparative Example 1 and Comparative Example 2, the gelatin aqueous solution was prepared according to the components and parts by weight shown in the following Table 1, and the gelatin microbead was prepared by the same process as in Example 1.

**[Table 1]**

| | First aminated gelatin (wt%) | Second aminated gelatin (wt%) | Fucoidan (wt%) | EGCG (wt%) |
|---|---|---|---|---|
| Example 1 | 35 | 15 | 7.5 | - |
| Example 2 | 35 | 15 | 5 | - |
| Example 3 | 35 | 15 | 2.5 | - |
| Example 4 | 35 | 15 | 1 | - |
| Example 5 | 10 | 40 | 7.5 | 0.5 |
| Example 6 | 10 | 40 | 7.5 | 0.25 |
| Example 7 | 15 | 35 | 7.5 | 0.5 |
| Example 8 | 15 | 35 | 7.5 | 0.25 |
| Comparative Example 1 | 50 | 0 | 0 | 0 |
| Comparative Example 2 | 15 | 35 | 0 | 0 |

**Test Example**

**Measurement of Iron Adsorption Rate**

About 100 mg of gelatin microbeads prepared according to Examples 1 to 8 was added into 10 ml of 0.018 M $FeCl_3$ aqueous solution and uniformly mixed. Then, the gelatin microbead was immersed in the $FeCl_3$ aqueous solution, and a supernatant of the $FeCl_3$ aqueous solution was collected every hour.

Then, the amount of iron contained in the supernatant was measured by ICP (Inductively Coupled Plasma), and the Fe concentration in the $FeCl_3$ aqueous solution was calculated based on the volume of the collected aqueous solution and the amount of iron. Therefore, an iron adsorption rate was calculated according to Equation 1.

The following Table 2 shows iron adsorption rates of respective Examples:
As shown in Table 2, the gelatin microbeads prepared according to Examples 1 to 8 have a high iron adsorption rate.

[**Table 2**]

| | Iron adsorption rate after 1 hour (%/100 mg) | Iron adsorption rate after 12 hours (%/100 mg) | Iron adsorption rate after 24 hours (%/100 mg) | Iron adsorption rate after 48 hours (%/100 mg) | Iron adsorption rate after 72 hours (%/100 mg) |
|---|---|---|---|---|---|
| Example 1 | 10.88 | 11.86 | 11.57 | 10.62 | 10.59 |
| Example 2 | 9.00 | 11.60 | 10.51 | 8.46 | 9.28 |
| Example 3 | 8.67 | 9.49 | 7.05 | 7.26 | 7.62 |

(continued)

| | Iron adsorption rate after 1 hour (%/100 mg) | Iron adsorption rate after 12 hours (%/100 mg) | Iron adsorption rate after 24 hours (%/100 mg) | Iron adsorption rate after 48 hours (%/100 mg) | Iron adsorption rate after 72 hours (%/100 mg) |
|---|---|---|---|---|---|
| Example 4 | 6.41 | 8.62 | 7.06 | 6.72 | 6.83 |
| Example 5 | 11.73 | 10.47 | 8.07 | 7.90 | 7.98 |
| Example 6 | 10.63 | 10.47 | 8.07 | 7.90 | 7.98 |
| Example 7 | 10.79 | 12.21 | 10.68 | 10.55 | 12.87 |
| Example 8 | 13.07 | 12.70 | 9.57 | 10.61 | 11.88 |
| Comparative Example 1 | No iron adsorption | | | | |
| Comparative Example 2 | No iron adsorption | | | | |

**Doxorubicin Conjugation Rate**

About 100 mg of gelatin microbeads prepared according to Examples 1 to 8 and Comparative Examples 1 and 2 was added into 10 ml of a doxorubicin solution having a concentration of about 1 mg/ml and uniformly mixed. Then, the gelatin microbead was immersed, and a supernatant of the doxorubicin solution was collected to measure the absorbance using light of a wavelength of about 550 nm.

The absorbance of each of Examples 1 to 8 and Comparative Examples 1 and 2 at a wavelength of 550nm was measured and the concentration of each was measured based on the calibration curve shown in **FIG. 2**, and the conjugation rate (%/100 mg) of doxorubicin adsorbed onto the gelatin microbead prepared according to each of Examples and Comparative Examples was calculated by comparison with the initial concentration.

**[Table 3]**

| | Absorbance | Concentration after adsorption (mg/mℓ) | Doxorubicin adsorption rate (%/100mg) |
|---|---|---|---|
| Example 1 | 0.6233 | 0.1956 | 84.44 |
| Example 2 | 0.7947 | 0.2509 | 74.91 |
| Example 3 | 1.1483 | 0.3651 | 63.49 |
| Example 4 | 2.8003 | 0.8986 | 10.14 |
| Example 5 | 1.0647 | 0.3381 | 66.19 |
| Example 6 | 0.1860 | 0.0544 | 94.56 |
| Example 7 | 0.4137 | 0.1279 | 87.21 |
| Example 8 | 0.6960 | 0.2191 | 78.09 |
| Comparative Example 1 | 3.107 | 0.9977 | 0.23 |
| Comparative Example 2 | 3.083 | 0.9899 | 1.01 |

As shown in Table 3, the gelatin microbeads prepared according to Examples 1 to 8 have a high doxorubicin conjugation rate.

**Particle Size Analysis**

The gelatin microbeads prepared according to Example were analyzed through a scanning electron microscope, and it can be seen that the prepared porous spherical gelatin microbeads have a uniform sponge structure with a particle size of from about 150 μm to about 355 μm.

**Claims**

1. An embolic microbead, comprising a biocompatible polymer,

   wherein the biocompatible polymer comprises a gelatin crosslinked with an iron adsorption block and,
   wherein the iron adsorption block includes a fucoidan and/or a polyphenol.

2. The embolic microbead of claim 1,

   wherein the polyphenol is a biodegradable anionic polyphenol, and
   wherein the polyphenol includes at least one selected from tannin, gallic acid, tannic acid, catechin, epicatechin, epigallocatechin gallate, quercetin, resveratrol, isoflavone, flavonoids, and polyphenols derived from seaweeds.

3. The embolic microbead of claim 1,

   wherein the gelatin is an aminated gelatin, and
   wherein the embolic microbead comprises cross-linking of a cationic aminated gelatin and an anionic fucodian.

4. The embolic microbead of claim 1,
   wherein a weight ratio of the gelatin : the iron adsorption block is 4:1 to 10:1.

5. The embolic microbead of claim 1,
   wherein the gelatin comprises a first gelatin having a first average molecular weight; and a second gelatin having a second average molecular weight smaller than the first average molecular weight.

6. The embolic microbead of claim 1,

   wherein an iron adsorption rate after 24 hours(IAd24) based on 100 mg of the embolic microbead, represented by the following Formula 1, is 5 %/100 mg or more:

   [Formula 1]

   $$IAd24 = \frac{(IC0-IC24)}{IC0} \times 100;$$

   wherein the IC0 is an initial molar concentration of a $FeCl_3$ aqueous solution, and
   wherein the IC24 is a molar concentration of an iron ion of the $FeCl_3$ aqueous solution left for 24 hours after adding the embolic microbead to the $FeCl_3$ aqueous solution.

7. The embolic microbead of claim 1,

   wherein an iron adsorption rate after 48 hours(IAd48) based on 100 mg of the embolic microbead, represented by the following Formula 2, is 5 %/100 mg or more:

   [Formula 2]

   $$IAd48 = \frac{(IC0 - IC48)}{IC0} \times 100;$$

   wherein the IC0 is an initial molar concentration of a $FeCl_3$ aqueous solution, and

wherein the IC48 is a molar concentration of an iron ion of the $FeCl_3$ aqueous solution left for 48 hours after adding the embolic microbead to the $FeCl_3$ aqueous solution.

**8.** An embolic microbead, comprising a biocompatible polymer according to claim 1,

wherein the biocompatible polymer comprises a gelatin crosslinked with an iron adsorption block,
wherein the iron adsorption block includes a fucoidan and/or a polyphenol, and
wherein the embolic microbead is combined with an anticancer drug.

**9.** The embolic microbead of claim 8,
wherein the anticancer drug is selected from doxorubicin, irinotecan, gemcitabine, oxaliplatin and docetaxel.

**10.** The embolic microbead of claim 8,

wherein a combining rate with the anticancer drug after 1 hour(AAd) based on 100 mg of the embolic microbead, represented by the following Formula 6, is 10 %/100 mg or more:

[Formula 6]

$$AAd = \frac{(AC0-AC1)}{AC0} \times 100;$$

wherein the AC0 is an initial mg/mℓ concentration of an anticancer drug solution, and
wherein the AC1 is a mg/mℓ concentration of the anticancer drug of the anticancer drug solution left for 1 hour after adding the embolic microbead to the anticancer drug solution.

**11.** A composition for treating proliferative diseases, comprising an embolic microbead,

wherein the embolic microbead comprises a gelatin crosslinked with an iron adsorption block,
wherein the iron adsorption block includes a fucoidan and/or a polyphenol, and
wherein the embolic microbead is combined with an iron component and/or an anticancer drug.

**12.** The composition for treating proliferative diseases of claim 11,
wherein the proliferative diseases include at least one selected from neovascularization, tumor, cancer, bone diseases, fibroplasia disorders, statomegaly, renal cyst, and atherosclerosis.

**13.** The composition for treating proliferative diseases of claim 12,
wherein the cancer includes at least one selected from solid tumor, hematologic malignancy, colorectal cancer, uterine cancer, uterine myoma, meningioma, lung cancer, small cell lung cancer, non-small cell lung cancer, gastrointestinal cancer, bowel cancer, breast cancer, ovarian cancer, prostate cancer, testicular cancer, liver cancer, renal cancer, bladder cancer, pancreatic cancer, sarcoma, osteosarcoma, kaposi's sarcoma and melanoma.

## Patentansprüche

**1.** Embolie-Mikrokügelchen, umfassend ein biokompatibles Polymer,

wobei das biokompatible Polymer eine mit einem Eisenadsorptionsblock vernetzte Gelatine umfasst, und
wobei der Eisenadsorptionsblock ein Fucoidan und/oder ein Polyphenol enthält.

**2.** Embolie-Mikrokügelchen nach Anspruch 1,

wobei das Polyphenol ein biologisch abbaubares anionisches Polyphenol ist, und
wobei das Polyphenol mindestens eines enthält, welches ausgewählt ist aus Tannin, Gallussäure, Tanninsäure, Catechin, Epicatechin, Epigallocatechingallat, Quercetin, Resveratrol, Isoflavon, Flavonoiden und aus Seetang stammenden Polyphenolen.

3. Embolie-Mikrokügelchen nach Anspruch 1,

wobei die Gelatine eine aminierte Gelatine ist, und
wobei das Embolie-Mikrokügelchen eine Vernetzung einer kationischen aminierten Gelatine und eines anionischen Fucoidans umfasst.

4. Embolie-Mikrokügelchen nach Anspruch 1,
wobei ein Gewichtsverhältnis Gelatine : Eisenadsorptionsblock 4:1 bis 10:1 beträgt.

5. Embolie-Mikrokügelchen nach Anspruch 1,
wobei die Gelatine eine erste Gelatine, die ein erstes mittleres Molekulargewicht aufweist; und eine zweite Gelatine, die ein zweites mittleres Molekulargewicht aufweist, das kleiner ist als das erste mittlere Molekulargewicht, umfasst.

6. Embolie-Mikrokügelchen nach Anspruch 1,

wobei eine Eisenadsorptionsrate nach 24 Stunden (IAd24) bezogen auf 100 mg des Embolie-Mikrokügelchens, dargestellt durch die folgende Formel 1, 5 %/100 mg oder mehr beträgt:

[Formel 1]

$$IAd24 = \frac{(IC0-IC24)}{IC0} \times 100;$$

wobei die IC0 eine anfängliche molare Konzentration einer wässrigen $FeCl_3$-Lösung ist, und
wobei die IC24 eine molare Konzentration eines Eisenions der wässrigen $FeCl_3$-Lösung ist, die 24 Stunden nach Zugabe des Embolie-Mikrokügelchens zu der wässrigen $FeCl_3$-Lösung verbleibt.

7. Embolie-Mikrokügelchen nach Anspruch 1,

wobei eine Eisenadsorptionsrate nach 48 Stunden (IAd48) bezogen auf 100 mg des Embolie-Mikrokügelchens, dargestellt durch die folgende Formel 2, 5 %/100 mg oder mehr beträgt:

[Formel 2]

$$IAd48 = \frac{(IC0 - IC48)}{IC0} \times 100;$$

wobei die IC0 eine anfängliche molare Konzentration einer wässrigen $FeCl_3$-Lösung ist, und
wobei die IC48 eine molare Konzentration eines Eisenions der wässrigen $FeCl_3$-Lösung ist, die 48 Stunden nach Zugabe des Embolie-Mikrokügelchens zu der wässrigen $FeCl_3$-Lösung verbleibt.

8. Embolie-Mikrokügelchen, umfassend ein biokompatibles Polymer nach Anspruch 1,

wobei das biokompatible Polymer eine mit einem Eisenadsorptionsblock vernetzte Gelatine umfasst,
wobei der Eisenadsorptionsblock ein Fucoidan und/oder ein Polyphenol enthält, und
wobei das Embolie-Mikrokügelchen mit einem Antikrebs-Medikament kombiniert ist.

9. Embolie-Mikrokügelchen nach Anspruch 8,
wobei das Antikrebs-Medikament ausgewählt ist aus Doxorubicin, Irinotecan, Gemcitabin, Oxaliplatin und Docetaxel.

10. Embolie-Mikrokügelchen nach Anspruch 8,

wobei eine Kombinationsrate mit dem Antikrebs-Medikament nach 1 Stunde (AAd) bezogen auf 100 mg des Embolie-Mikrokügelchens, dargestellt durch die folgende Formel 6, 10 %/100 mg oder mehr beträgt:

[Formel 6]

$$AAd = \frac{(AC0-AC1)}{AC0} \times 100$$;

wobei die AC0 eine anfängliche Konzentration in mg/ml einer Antikrebs-Medikament-Lösung ist, und
wobei die AC1 eine Konzentration in mg/ml des Antikrebs-Medikaments der Antikrebs-Medikament-Lösung ist, die 1 Stunde nach Zugabe des Embolie-Mikrokügelchens zu der Antikrebs-Medikament-Lösung verbleibt.

**11.** Zusammensetzung zur Behandlung proliferativer Erkrankungen, umfassend ein Embolie-Mikrokügelchen,

wobei das Embolie-Mikrokügelchen eine mit einem Eisenadsorptionsblock vernetzte Gelatine umfasst,
wobei der Eisenadsorptionsblock ein Fucoidan und/oder ein Polyphenol enthält, und
wobei das Embolie-Mikrokügelchen mit einer Eisenkomponente und/oder einem Antikrebs-Medikament kombiniert ist.

**12.** Zusammensetzung zur Behandlung proliferativer Erkrankungen nach Anspruch 11,
wobei die proliferativen Erkrankungen mindestens eines umfassen, welches ausgewählt ist aus Neovaskularisation, Tumor, Krebs, Knochenerkrankungen, Fibroplasie-Störungen, Statomegalie, Nierenzyste und Atherosklerose.

**13.** Zusammensetzung zur Behandlung proliferativer Erkrankungen nach Anspruch 12,
wobei der Krebs mindestens eines umfasst, welches ausgewählt ist aus solidem Tumor, bösartiger hämatologischer Erkrankung, kolorektalem Krebs, Gebärmutterkrebs, Gebärmuttermyom, Meningeom, Lungenkrebs, kleinzelligem Lungenkrebs, nicht-kleinzelligem Lungenkrebs, gastrointestinalem Krebs, Darmkrebs, Brustkrebs, Eierstockkrebs, Prostatakrebs, Hodenkrebs, Leberkrebs, Nierenkrebs, Blasenkrebs, Bauchspeicheldrüsenkrebs, Sarkom, Osteosarkom, Kaposi-Sarkom und Melanom.

## Revendications

**1.** Microbille embolique, comprenant un polymère biocompatible,

dans laquelle le polymère biocompatible comprend une gélatine réticulée avec un bloc d'adsorption de fer et,
dans laquelle le bloc d'adsorption de fer comprend un fucoïdan et/ou un polyphénol.

**2.** Microbille embolique selon la revendication 1,
dans laquelle le polyphénol est un polyphénol anionique biodégradable et dans laquelle le polyphénol compred au moins un choisi parmi le tannin, l'acide gallique, l'acide tannique, la catéchine, l'épicatéchine, le gallate d'épigallocatéchine, la quercétine, le resvératrol, une isoflavone, des flavo-noïdes et des polyphénols dérivés d'algues.

**3.** Microbille embolique selon la revendication 1,

dans laquelle la gélatine est une gélatine aminée et
dans laquelle la microbille embolique comprend la réticulation d'une gélatine aminée cationique et d'un fucodian anionique.

**4.** Microbille embolique selon la revendication 1,
dans laquelle un rapport en poids de la gélatine : bloc d'adsorption de fer est de 4:1 à 10:1.

**5.** Microbille selon la revendication 1,
dans laquelle la gélatine comprend une première gélatine ayant un premier poids moléculaire moyen ; et une seconde gélatine ayant un second poids moléculaire moyen plus faible que le premier poids moléculaire moyen.

**6.** Microbille embolique selon la revendication 1,

dans laquelle un taux d'adsorption de fer après 24 heures (IAd24) sur la base de 100 mg de la microbille embolique, représentée par la formule 1 suivante, est de 5 %/100 mg ou plus :

[Formule 1]

$$IAd24 = \frac{(IC0-IC24)}{IC0} \times 100;$$

où l'IC0 est une concentration molaire initiale d'une solution aqueuse de $FeCl_3$ et
où l'IC24 est une concentration molaire d'un ion de fer de la solution aqueuse de $FeCl_3$ laissée durant 24 heures après l'addition de la microbille embolique à la solution aqueuse de $FeCl_3$.

**7.** Microbille embolique selon la revendication 1,

dans laquelle un taux d'adsorption de fer après 48 heures (IAd48) sur la base de 100 mg de la microbille embolique, représenté par la formule 2 suivante, est de 5 %/100 mg ou plus ;

[Formule 2]

$$IAd48 = \frac{(IC0 - IC48)}{IC0} \times 100;$$

où l'IC0 est une concentration molaire initiale d'une solution aqueuse de $FeCl_3$ et
où l'IC48 est une concentration molaire d'un ion de fer de la solution aqueuse de $FeCl_3$ laissée durant 48 heures après l'addition de la microbille embolique à la solution aqueuse de $FeCl_3$.

**8.** Microbille embolique, comprenant un polymère biocompatible selon la revendication 1,

dans laquelle le polymère biocompatible comprend une gélatine réticulée avec un bloc d'adsorption de fer,
dans laquelle le bloc d'adsorption de fer comprend un fucoïdan et/ou un polyphénol, et
dans laquelle la microbille embolique est combinée avec un médicament anticancéreux.

**9.** Microbile embolique selon la revendication 1,
dans laquelle le médicament anticancéreux est choisi parmi la doxorubicine, l'irinotécan, la gemcitabine, l'oxaliplatine et le docétaxel.

**10.** Microbille embolique selon la revendication 8,

dans laquelle un taux de combinaison avec le médicament anticancéreux après 1 heure (AAd) sur la base de 100 mg de la microbille embolique, représenté par la formule 6 suivante, est de 10 %/100 mg ou plus :

[Formule 6]

$$AAd = \frac{(AC0-AC1)}{AC0} \times 100;$$

où l'AC0 est une concentration initiale en mg/ml d'une solution de médicament anticancéreux et
où l'AC1 est une concentration en mg/ml du médicament anticancéreux de la solution de médicament anticancéreux laissée durant 1 heure après l'addition de la microbille embolique à la solution de médicament anticancéreux.

**11.** Composition pour traiter les maladies prolifératives, comprenant une microbille embolique,

dans laquelle la microbille embolique comprend une gélatine réticulée avec un bloc d'adsorption de fer,
dans laquelle le bloc d'adsorption de fer comprend un fucoïdan et/ou un polyphénol, et
dans laquelle la microbille embolique est combinée avec un composant de fer et/ou un médicament anticancéreux.

**12.** Composition pour traiter les maladies prolifératives selon la revendication 11,
dans laquelle les maladies prolifératives comprennent au moins un choisi parmi la néovascularisation, une tumeur, un cancer, les maladies osseuses, les troubles de fibroplasie, la statomégalie, le kyste rénal, et l'athéroclérose.

**13.** Composition pour traiter les maladies prolifératives selon la revendication 12,
dans laquelle le cancer comprend au moins un choisi parmi une tumeur solide, une malignité hématologique, le cancer colorectal, le cancer utérin, le myome utérin, le méningiome, le cancer du poumon, le cancer du poumon à petites cellules, le cancer du poumon non à petites cellules, le cancer gastrointestinal, le cancer de l'intestin, le cancer du sein, le cancer ovarien, le cancer de la prostate, le cancer testiculaire, le cancer du foie, le cancer rénal, le cancer de la vessie, le cancer pancréatique, le sarcome, l'ostéosarcome, le sarcome de kaposi, et le mélanome.

[FIG 1]

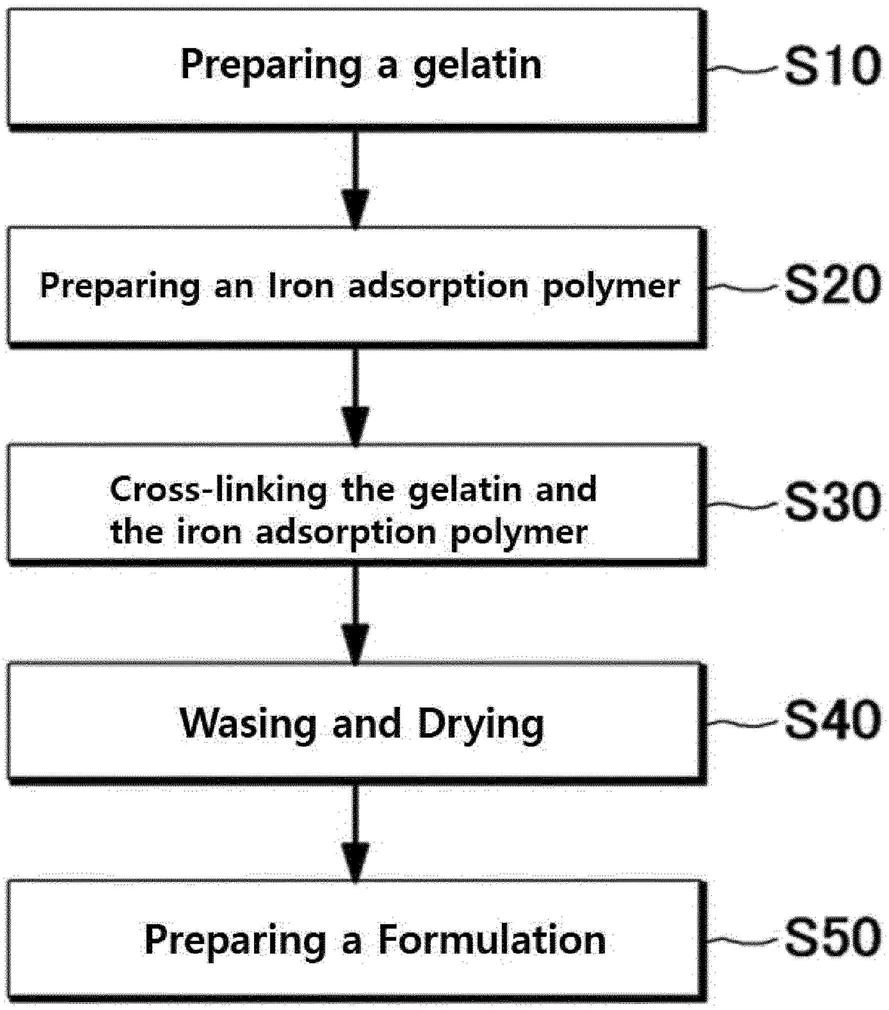
Preparing a gelatin
S10
Preparing an Iron adsorption polymer
S20
Cross-linking the gelatin and
the iron adsorption polymer
S30
Wasing and Drying
S40
Preparing a Formulation
S50

[FIG 2]

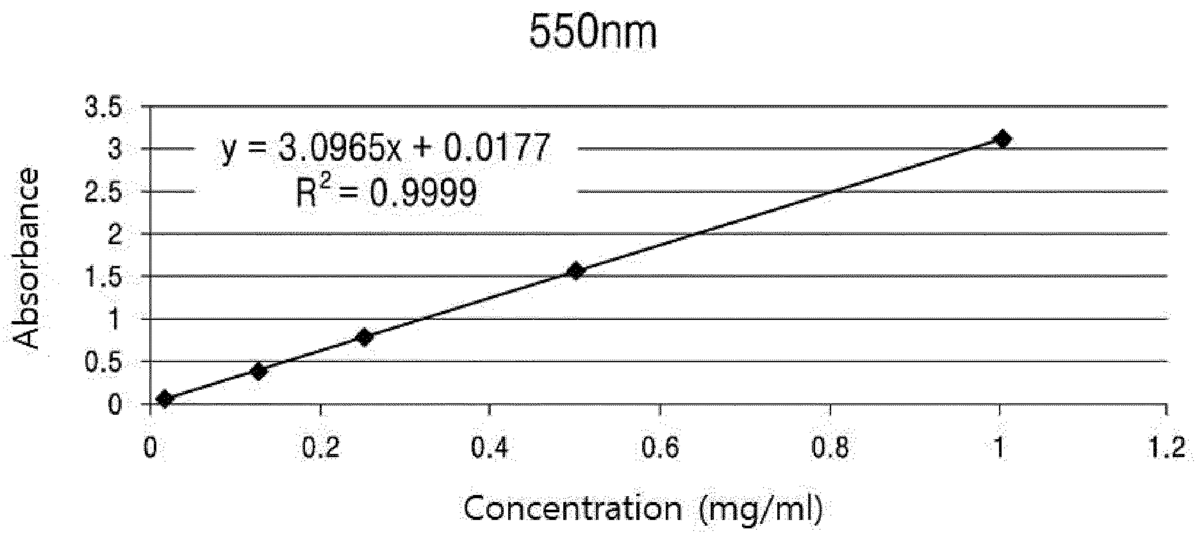
550nm
y = 3.0965x + 0.0177
R2 = 0.9999
3.5
3
2.5
2
1.5
1
0.5
0
Absorbance
0
0.2
0.4
0.6
0.8
1
1.2
Concentration (mg/ml)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020010068458 A
- EP 2351779 A